# EUROPEAN PATENT SPECIFICATION

(11) **EP 4 164 728 B1**
(45) Date of publication and mention of the grant of the patent: **19.11.2025**
(21) Application number: 21821830.3
(22) Date of filing: 09.06.2021
(51) Int. Cl.: A61K 9/00, A61K 47/34, A61M 37/00, A61K 45/00, A61M 35/00, A61K 35/17

(54) **MICRONEEDLE PATCH FOR IN-SITU SEEDING OF CELLS**
MIKRONADELPFLASTER ZUR IN-SITU-IMPFUNG VON ZELLEN
TIMBRE À MICRO-AIGUILLES POUR ENSEMENCEMENT IN SITU DE CELLULES

(30) Priority: 12.06.2020 US 202063038724 P
(43) Date of publication of application: 19.04.2023
(73) Proprietor: The Regents of the University of California, Oakland, CA 94607-5200 (US)
(72) Inventor: GU, Zhen, Los Angeles, California 90095-7191 (US); LI, Hongjun, Los Angeles, California 90095-7191 (US)
(74) Representative: Ipsilon Benelux
(86) International application number: PCT/US2021/036673
(87) International publication number: WO 2021/252673

(56) References cited:
- WO-A1-2007/042818
- WO-A1-2018/017674
- WO-A1-2020/041694
- WO-A1-2020/041694
- WO-A1-2020/092229
- US-A1- 2013 158 505
- US-A1- 2014 336 487
- US-A1- 2017 157 381
- US-A1- 2019 046 479

## Description

### Technical Field

The technical field generally relates to a patch that incorporates microneedles for the delivery of cells in mammalian tissue including, but not limited to, chimeric antigen receptor T cells (CAR T) in solid tumors.

### Background

Chimeric antigen receptor (CAR) expressing T cells engineered with specific tumor-associated antigen (TAA) targeting ability have shown remarkable potency in hematological malignancies. The U.S. Food and Drug Administration (FDA) approved the use of CD19-targeting CAR T cells therapy for treating pediatric acute lymphoblastic leukemia in 2017. In contrast, clinical evaluation using CAR T cells for solid tumors did not show remarkable antitumor effects, yet. Solid tumors are characterized by unique microenvironment (TME) with both physical and physiochemical barriers that hamper the anti-tumor effects of CAR T cells. This includes, for example, abnormal vasculature, dense extracellular matrix and stroma, and interstitial fluid pressure and physical barriers preventing CAR T cells from infiltrating the tumor bed. Furthermore, immunosuppressive cells and soluble factors in TME hamper proliferation and effector function of CAR T cells.

Post-surgical *in situ* administration of CAR T cells offers a potential solution for overcoming the physical barriers in solid tumors. Surgical removal of bulky tumors delays tumor recurrence, relieves physical barriers, and expose residual cancer cells to endogenous effector T cells. However, the local spread of residual micro-tumors after surgery poses serious obstacles to precise delivery of adoptively transferred CAR T cells. Other treatment modalities are needed.

Each of WO 2007/042818 A1, WO 2020/041694 A1 and WO 2018/017674 A1 discloses a porous microneedle patch for the localized delivery of cells to living tissue comprising a base having a plurality of microneedles extending away from the surface of the base, wherein the plurality of microneedles comprises a polymer having pores formed therein, wherein the pores contain cells therein. While this provides technical advances, improvements remain desirable.

### Summary

The invention is directed to a porous microneedle patch for the localized delivery of live chimeric antigen receptor T (CAR T) cells to living tissue, according to claim 1. Further developments are according to dependent claims 2-3.

The invention is also directed to a method of manufacturing a porous microneedle patch for the localized delivery of living cells to living tissue, according to claim 4. Further developments are according to dependent claims 5-11.

The porous microneedle patch may be used by applying the patch (containing living cells) to mammalian tissue. The tissue may include diseased or non-diseased tissue. The porous microneedle patch (which contains CAR T cells) may be applied, for example, to tissue adjacent to or surrounding a region where a tumor has been resected or excised from living tissue. The porous microneedle patch may also be applied directly to the tumor tissue. One particular application is the use of CAR T cells but other cell types may be loaded into the porous microneedle patch and delivered to tissue.

### Brief Description of the Drawings

FIG. 1A illustrates a plan view of a patch for the delivery of CAR T cells according to one embodiment. The patch includes a base or substrate and a plurality of microneedles that extend from a surface thereof. An optional backing is also illustrated.
FIG. 1B illustrates a cross-sectional view of a patch illustrating the base or substrate and the plurality of microneedles that extend from a surface thereof. CAR T cells are illustrated being disposed within the microneedles and the base or substrate. The optional backing is also seen in FIG. 1B.
FIG. 1C illustrates a patch for transdermal therapeutic agent delivery being applied to tissue of a mammal (e.g., human).
FIG. 1D illustrates a plan view of an alternative embodiment of a patch for the delivery of CAR T cells.
FIG. 2A illustrates a schematic of porous microneedle patch (PMN) fabrication including the etching of sacrificial microparticles to form pores in the microneedles of the porous microneedle patch.
FIG. 2B schematically illustrates CAR T cell loading of the porous microneedle patch and application of CAR T cell-loaded PMN (PMN@CAR T) to the tumor bed after surgery.
FIG. 2C is a representative photograph of a porous microneedle patch, scale bar: 2 mm.
FIG. 2D is a representative SEM images of the PMN tip showing the porous structure, scale bar: 50 µm.
FIG. 2E is a representative SEM images of the cross section of PMN with different layer widths, scale bar: 20 µm.
FIG. 2F is a 3D reconstruction of the PMN@CAR T confocal images. CAR T cells were labelled with CFSE and the microneedle patch was labelled with rhodamine B, respectively, scale bar: 100 µm.

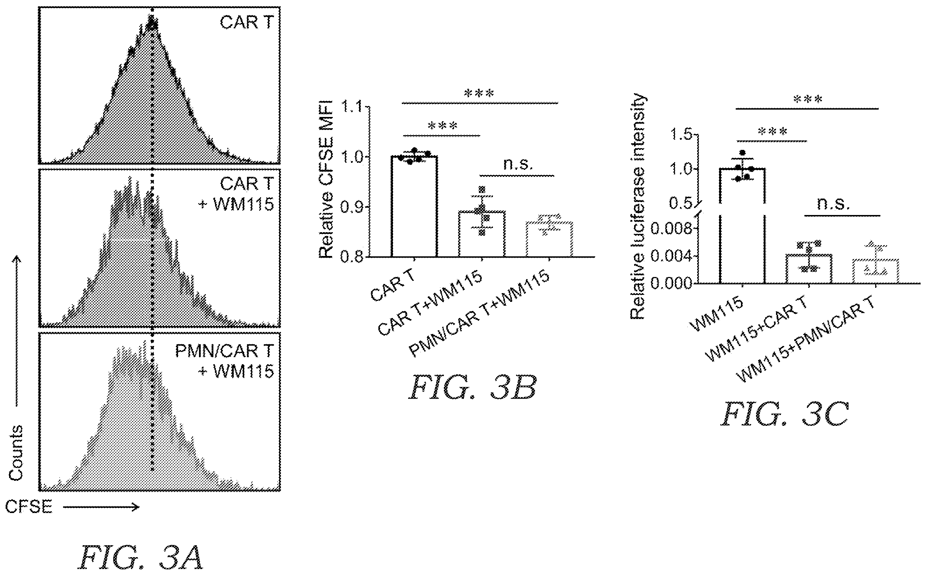

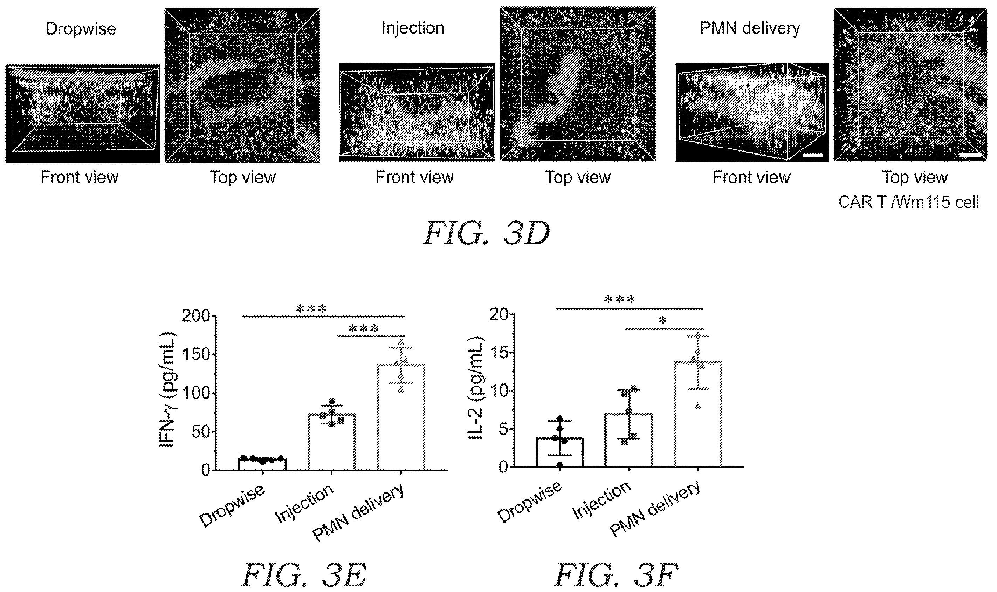

FIGS. 3A-3F: PMN promotes CAR T cell infiltration in a 3D tumor model *in vitro.* FIG. 3A: CSPG4⁺ CAR T cells labelled with CFSE were cocultured for three days with CSPG4-expressing WM115 tumor cells. Representative flow cytometry histograms showing CFSE dilution are presented. FIG. 3B: Relative mean fluorescence intensity of CFSE in (FIG. 3A), indicating T cell proliferation, n = 5. FIG. 3C: CSPG4⁺ CAR T cells were cocultured for three days with Firefly luciferase labels CSPG4-expressing WM115 tumor cells. Relative luciferase intensity after three days of culture is illustrated, n = 5. FIG. 3D: Schematic of three approaches (dropwise, single needle injection and PMN) used to deliver CAR T cells into a 3D tumor model *in vitro.* The 3D reconstruction illustrates CAR T cells and WM115 cancer cells distribution in the 3D tumor model at day three after T cells application, scale bar: 300 µm. Cytokine level of human IFN-y (FIG. 3E) and IL-2 (FIG. 3F) after T cell application into the 3D tumor model at day three, n = 5. Data are presented as mean ± SD, statistical significance was calculated *via* one-way ANOVA with a Tukey post-hoc test. *P* value: **P <* 0.05; ***P* < 0.01; ****P* < 0.001, n.s. means no significant difference.

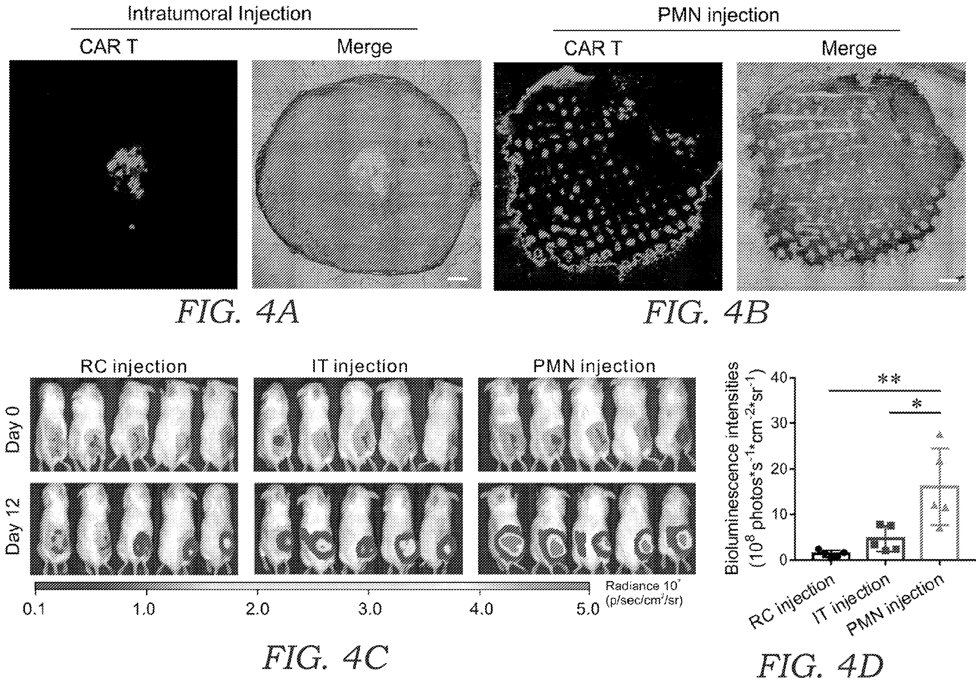

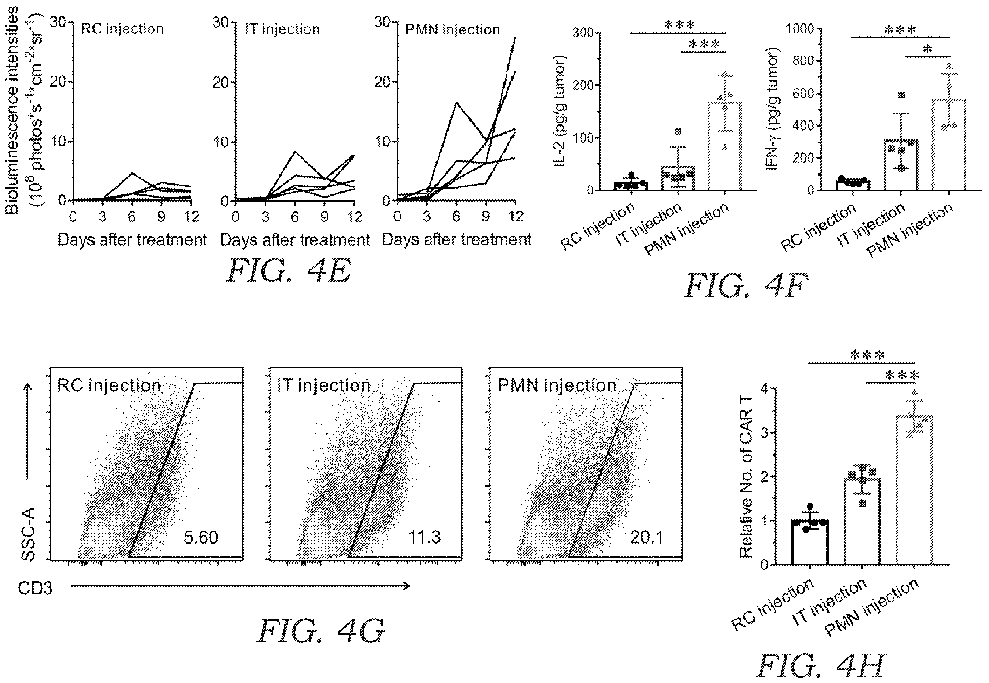

FIGS. 4A-4H: PMN promotes wider distribution of CAR T cells *in vivo.* Distribution of CAR T cells within the WM115 tumor after (FIG. 4A) single needle intratumoral injection and (FIG. 4B) PMN insertion, scale bar: 1 mm. CAR T cells were pre-labelled with DIR. FIG. 4C: T cell bioluminescence of luciferase-expressing CAR T cells inoculated within the post-surgical tumor bed. CAR T cells were delivered directly in the resection cavity (RC injection), intratumorally using a single needle (IT injection) or intratumorally via porous microneedle assisted implant (PMN injection). FIG. 4D: T cell bioluminescence at day 12 after application is illustrated, n = 5. FIG. 4E: Kinetics of T cell bioluminescence in (FIG. 4C). FIG. 4 F: Levels of human IL-2 and IFN-*γ* detected with the tumor lysates at day 12 days after CAR T cell treatment, n = 5. Representative flow cytometric plots (FIG. 4G) and quantification (FIG. 4H) of CAR T cells (CD3 positive) within the tumor at day 12 after CAR T cell administration, n = 5. Data are presented as mean ± SD, statistical significance was calculated *via* one-way ANOVA with a Tukey post-hoc test. *P* value: **P* < 0.05; ****P* < 0.001.

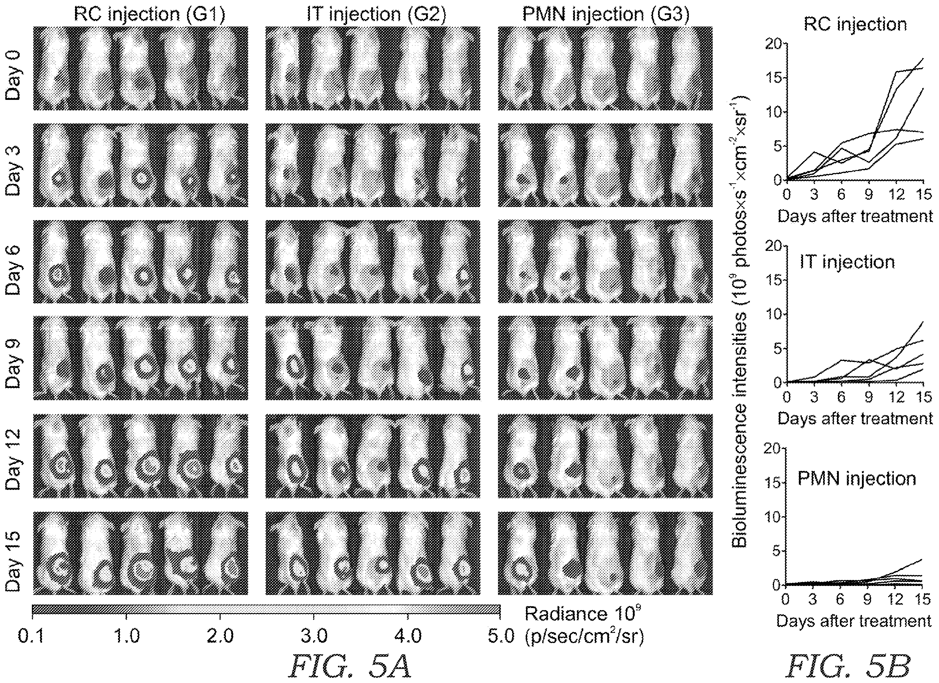

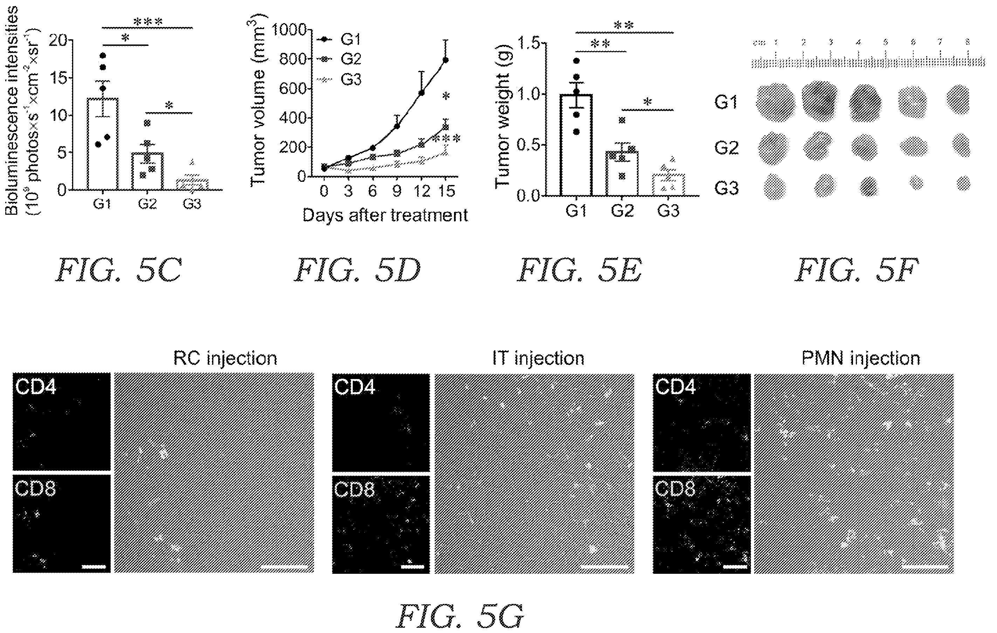

FIGS. 5A-5G: CAR T cells delivered by PMN show ehanced antitumor effects. FIG. 5 A: Representative tumor bioluminescence of WM115-bearing mice treated with CAR T cells administared with the resection cavity via RC injection, IT injection or PMN injection. (FIG. 5B) Kinetics of tumor bioluminescence and (FIG. 5C) tumor biolumineswcenc at day 15 post-treatment. FIG. 5D: Kinetics of tumor growth measured using a caliper, (FIG. 5E) weight and (FIG. 5F) the size of the removed tumors after treatment, n = 5. (FIG. 5G) Representative immunofluorescence showing CD4⁺ and CD8⁺ T cells withi the tumor after treatment, scale bar 100 µm. Data are presented as mean ± SD, statistical significance was calculated *via* one-way ANOVA with a Tukey post-hoc test. *P* value: **P* < 0.05; ***P* < 0.01; ****P* < 0.001.
FIG. 6 illustrates the ¹H NMR spectra of the 4-arm-PLGA. The molecular structure of 4-arm-PLGA is also illustrated.
FIG. 7A illustrates the SEM image of the CaCO₃ microparticles, scale bar: 10 µm.
FIG. 7B illustrates the size distribution of CaCO₃ microparticles.
FIG. 8 illustrates an image of CaCO₃ containing microneedle patch before (left) and after (right) swelling in 1,4-dioxane.
FIG. 9 illustrates a SEM image of a porous microneedle patch; scale bar: 200 µm.
FIG. 10 shows SEM images showing the cross sections of the unetched porous microneedle; scale bar: 20 µm.
FIG. 11 illustrates graphs of mechanical strength of unetched solid microneedle (SMN), porous microneedle (PMN) and CAR T cells loaded PMN (PMN@CAR T).
FIGS. 12A is a representative flow cytometry quantification of CAR T cells calculated with counting beads.
FIG. 12B is a histogram showing the loading amount and loading efficiency of CAR T cells in the porous microneedle patch.
FIG. 13 illustrates flow cytometry analysis of CSPG4 expressed on WM115 cells.
FIG. 14A illustrate cytokine level of human IFN-*γ* after T cells co-incubation with WM115 cells in different conditions, n = 5. Data are presented as mean ± SD, statistical significance was calculated *via* one-way ANOVA with a Tukey post-hoc test. *P* value: ****P* < 0.001, n.s. means no significant difference.
FIG. 14B illustrate cytokine level of human IL-2 after T cells co-incubation with WM115 cells in different conditions, n = 5. Data are presented as mean ± SD, statistical significance was calculated *via* one-way ANOVA with a Tukey post-hoc test. *P* value: ****P* < 0.001, n.s. means no significant difference.
FIG. 15 illustrates bioluminescence images of mice administered with luciferase-expressing CAR T cells at the tumor site on day 3, day 6 and day 9 post-surgery.
FIG. 16 illustrates representative flow cytometry of CD3 positive T cells in peripheral blood 12 days after treatment with CAR T cells, n = 5. Histogram on right shows % of CAR T cells in peripheral blood for RC injection, IT injection, and porous microneedle patch (PMN) injection. Data are presented as mean ± SD, statistical significance was calculated *via* one-way ANOVA with a Tukey post-hoc test. *P* value: ****P* < 0.001.
FIG. 17 illustrates representative immunofluorescence images of tumors showing apoptosis cells after treatment with TUNEL, scale bar: 100 µm.

### Detailed Description of Illustrated Embodiments

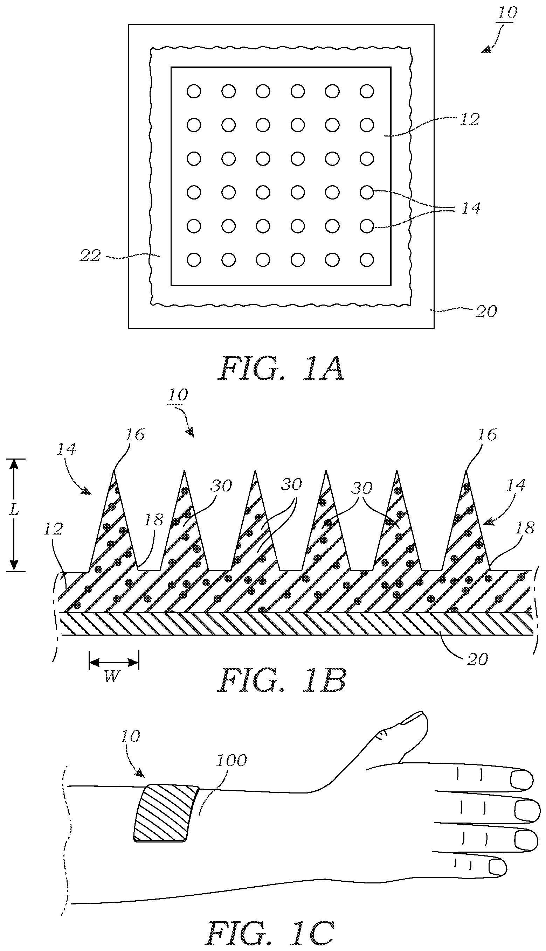

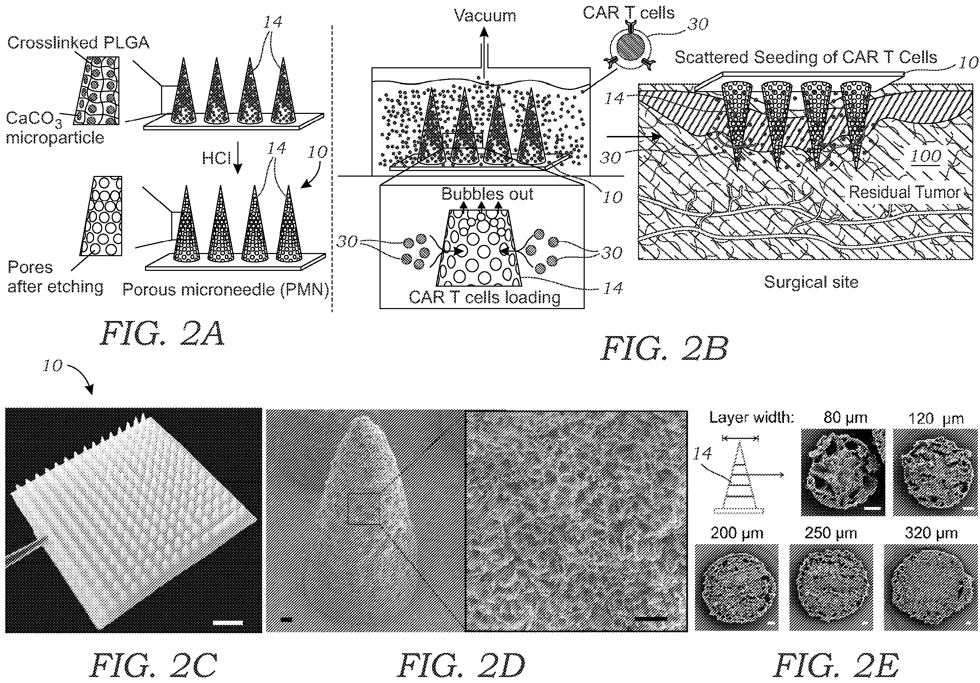

FIGS. 1A and 1B illustrates a porous microneedle patch 10 (sometimes also referred to herein as PMN) for the delivery of live cells 30 (seen in FIG. 1B) to mammalian tissue 100 as illustrated in FIG. 1C. In one particular embodiment, the cells 30 that are delivered are CAR T cells. However, in other embodiments, the cells 30 may include stem cells such as pluripotent stems cells for tissue regeneration. Other cells 30 include dendritic cells for immunotherapy applications. The mammalian tissue 100 may include diseased tissue such as solid tumor tissue. The tissue 100 may also include healthy tissue as well. In one embodiment, the porous microneedle patch 10 may be applied to tissue that is around or adjacent to diseased tissue (e.g., surrounding the tumor margin) which may contain residual tumor cells (e.g., where the cells 30 are CAR T cells). FIG. 2C illustrates a photographic image of one example of a porous microneedle patch 10.

As seen in FIGS. 1A and 1B, the porous microneedle patch 10 includes a base or substrate 12 that includes a plurality of microneedles 14 that extend or project from the substrate 12. The porous microneedle patch 10 or components thereof (e.g., microneedles 14 and/or base or substrate 12) may in some embodiments be partly or entirely biodegradable. The plurality of microneedles 14 generally extend or project in a perpendicular direction from a surface of the base or substrate 12 (seen in FIG. 1B). The plurality of microneedles 14 may be arranged in a regular repeating array as illustrated in FIG. 1A or, alternatively, they may be arranged in a random pattern. In one embodiment, the plurality of microneedles 14 that are formed on the base or substrate 12 may have substantially similar shapes and sizes. However, in other embodiments, the plurality of microneedles 14 may have different shapes and/or sizes. For example, the perimeter region of the array or field of microneedles 14 that extend from the base or substrate 12 may be longer or have different shapes than those in the central region of the porous microneedle patch 10 to better secure the porous microneedle patch 10 to site of application. Other configurations are possible as well.

In one particular embodiment, the microneedles 14 have a needle-like shape. For example, the microneedles 14 may include a sharpened tip 16 (seen in FIG. 1B) that aid in penetrating the tissue 100 (seen in FIG. 1C). The length (L) of the microneedles 14 may vary although typically the microneedles 14 extend less than about 2.0 mm from the base or substrate 12 (FIG. 1B). A typical length of the microneedles 14 is around 500-1,500 µm, although the dimensions may extend outside this range. The base 18 of the microneedle 14 is wider than the tip 16. Typically, the base 18 of the microneedle 14 may have a diameter or width (W) that is less than about 600 µm (e.g., 250 µm base and a height of around 1,500 µm) (FIG. 1B). The microneedles 14 may be separated from one another by a pitch or spacing that may be several hundred µm (e.g., 200 µm). The particular numbers, density, dimensions, and shape(s) of the microneedles 14 are controlled by the particular construction of the mold that is used to form the porous microneedle patch 10, which is described more in detail below.

Still referring to FIGS. 1A and 1B, the base or substrate 12 which holds the microneedles 14 may be optionally bonded or otherwise adhered to a backing material 20 (e.g., through the use of an adhesive, chemical linking, or the like). The backing material 20 may be made from a woven fabric, a plastic material such as polyvinylchloride, polyethylene, or polyurethane, or latex. The backing material 20 may be flexible so that the porous microneedle patch 10, when applied, can conformally cover the tissue 100 (seen in FIG. 1C). Optionally, the backing material 20 may include an adhesive material 22 that covers all or a portion of the tissue-facing surface of the backing material 20. For example, adhesive may be formed on the backing material 20 around the periphery of the base or substrate 12 or the backing material 20 so that the base or substrate 12 may be secured in place to the surface of the tissue 100. The adhesive material 22 aids in securing the porous microneedle patch 10 to the tissue 100. The adhesive material 22 may include resins (e.g., vinyl resins), acrylates such as methacrylates epoxy diacrylates. Alternatively, the porous microneedle patch 10 may be secured to the tissue 100 via engagement of the microneedles 14 in the tissue 100. In still other alternatives, the porous microneedle patch 10 may be secured to the tissue 100 using an adhesive or glue located on the base or substrate 12. A separate bandage or wrap may also be used to secure the porous microneedle patch 10 to the tissue 100.

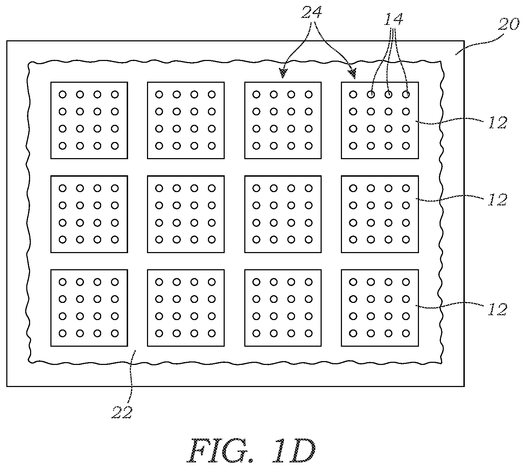

In one alternative embodiment which is illustrated in FIG. 1D, multiple sub-patches 24 may be integrated into the backing material 20 to make the final porous microneedle patch 10. This may be useful for large coverage areas or curved surfaces that may pose a risk of breakage to the base or substrate 12. The various sub-patches 24, while having some rigidity, are still able to conform to the surface of the tissue 100 (e.g., FIG. 1C) due the flexible backing material 20 which enables bending of the overall porous microneedle patch 10. Because individual sub-patches 24 are smaller in size these do not experience significant bending stresses which would otherwise cause a larger, rigid structure to break in response to bending and/or manipulation. Bending or flexing can occur within the backing material 20 between the locations of where the sub-patches 24 are located (e.g., between the rows and columns of sub-patches 24).

The porous microneedle patch 10 is fabricated as a porous structure to accommodate the cells 30 as seen in FIG. 1B. The pores 28 in the porous microneedle patch 10 may be formed in at least the microneedles 14 but may also be present in the base 12. The pores 28 in the porous microneedle patch 10 may be formed by selective removal or etching of sacrificial microparticles that are temporarily contained in the porous microneedle patch 10 during the fabrication process. In one embodiment, the sacrificial microparticles include CaCO₃ microparticles that are then etched away to leave pores 28 inside the microneedles 14 and/or base 12. Other sacrificial microparticles that may be used to form the pores 28 including other carbonates, inorganic microparticles such as, for instance, magnesium carbonate, manganese carbonate, barium carbonate, sodium chloride, and calcium chloride.

For example, in one embodiment, the porous microneedle patch 10 is fabricated by molding and polymerizing the mixture of methacryloyl chloride modified 4-arm-poly (lactic-co-glycolic acid) (4-arm-PLGA), triethylene glycol diacetate, azobisisobutyronitrile (AIBN), along with CaCO₃ microparticles. While a 4-arm PLGA was used herein it should be appreciated that a different methacryloyl chloride modified multi-arm PLGA may be used. A high molecular weight linear PLGA may be optionally added to control the viscosity of the mixture to ensure homogeneous distribution of the CaCO₃ microparticles. AIBN acts as an initiator for crosslinking, which generates free radicals when heated to start the polymerization of methacryloyl chloride modified PLGA and triethylene glycol diacetate (TEGDA). TEGDA is used for improving the crosslinking efficiency. Additional polymer materials that may be used as an alternative to PLGA for the porous microneedle patch 10 include polyvinylpyrrolidone (PVP), polyvinyl alcohol (PVA), polycaprolactone (PCL), polylactic acid (PLA), or chitosan. It should be appreciated that other initiators or crosslinking agents may be needed in the polymerization of these polymer materials. In addition, polymerization may be aided by the application of heat or light. Other viscosity modifying agents that are appropriate for the particular polymer system may be added to aid in the distribution of the sacrificial microparticles.

The method of manufacturing a porous microneedle patch 10 for the localized delivery of living cells 30 to living tissue 100 includes providing a mold containing a plurality of needle-shaped cavities therein. Next, a solution containing a polymerizable polymer, an initiator or crosslinking agent, and sacrificial microparticles are then deposited onto to the mold. The solution is then crosslinked to create the patch structure (at this point it is not yet porous). The now-formed patch structure is then removed from the mold and is placed into a swelling solution which causes the patch to swell. The swelled patch is then placed in an etching solution to remove the sacrificial microparticles and form pores 28 in the porous microneedle patch 10. The porous microneedle patch 10 is then loaded with cells 30. This is done by exposing the porous microneedle patch 10 to a solution that contains the cells 30. In one embodiment, the porous microneedle patch 10 and solution is exposed to a vacuum which pulls gas out of the porous microneedle patch 10 and allow the intrusion of cells 30 into the pores 28.

In the embodiment that uses CaCO₃ microparticles as the sacrificial microparticles such as illustrated in FIG. 2A, following crosslinking of the microneedles 14 and/or base 12, the CaCO₃ microparticles are then removed (i.e., etched away) by swelling the porous microneedle patch 10 and exposing the CaCO₃ microparticles to an etching solution (e.g., hydrogen chloride dioxane solution). The size of the pores 28 in the porous microneedle patch 10 may have an average diameter within the size range of about 5-20 µm which can readily accommodate the CAR T cells 30 (having sizes within the range of about 5-10 µm). Of course, as explained herein, other types of cells 30 may also be loaded into the porous microneedle patch 10. Once the porous microneedle patch 10 is formed, the porous microneedle patch 10 is loaded with the CAR T cells 30. The loading of the CAR T cells 30 may occur under vacuum to aid in loading the cells therein. That is to say, the porous microneedle patch 10 is exposed to a solution that contains the cells 30. A vacuum is then applied to the porous microneedle patch 10 which removes gas contained in the porous microneedle patch 10 in form of bubbles (FIG. 2B). Cells 30 are then able to infiltrate the microneedle 14 within the pores 28 (illustrated in FIG. 2B).

As explained herein, the porous microneedle patch 10 offers a multipoint, scattered delivery of CAR T cells 30 seeding that promotes enhanced T cell infiltration within the tumor bed by overcoming poor T cell biodistribution caused by physical barriers in solid tumors. The porous microneedle patch 10 can be applied directly to tumors. Alternatively, the porous microneedle patch 10 can be applied to the resection cavity post-surgery to prevent local tumor recurrence and metastatic dissemination and offer a platform for living cell 30 delivery targeting a variety of diseases.

The porous microneedle patch 10 may be pre-loaded with the cells 30 which can then be used by the physician or other healthcare provider. In this embodiment, the porous microneedle patch 10 with the cells 30 may be stored in a solution containing cellular growth media and appropriate buffers. Alternatively, the porous microneedle patch 10 may be provided without the cells 30 being loaded therein. In this alternative, the cells 30 are added to the porous microneedle patch 10 on site (e.g., at the hospital, physician's office or other medical facility). For example, prior to use of the porous microneedle patch 10, the swelled porous microneedle patch 10 may be loaded with cells 30. The choice of cells 30 to be loaded into the porous microneedle patch 10 is made by the physician or other healthcare provider and is made based on the particular application for the porous microneedle patch 10. For example, for cancer treatment, CAR T cells 30 may be loaded into porous microneedle patch 10. Once the cells 30 are loaded into the porous microneedle patch 10, the porous microneedle patch 10 can then be applied to the tissue 100.

### Experimental

### Results

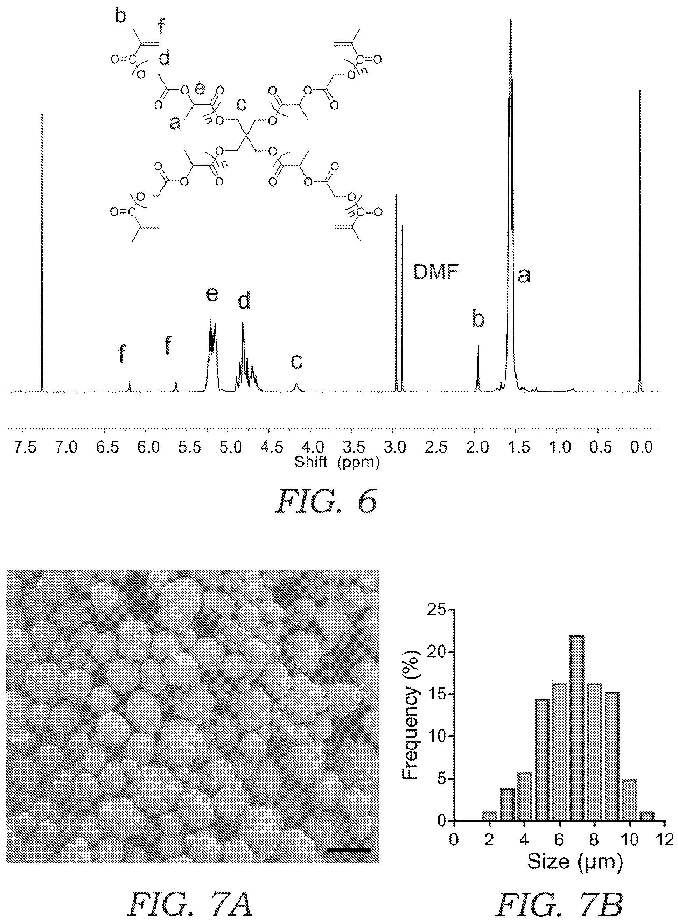

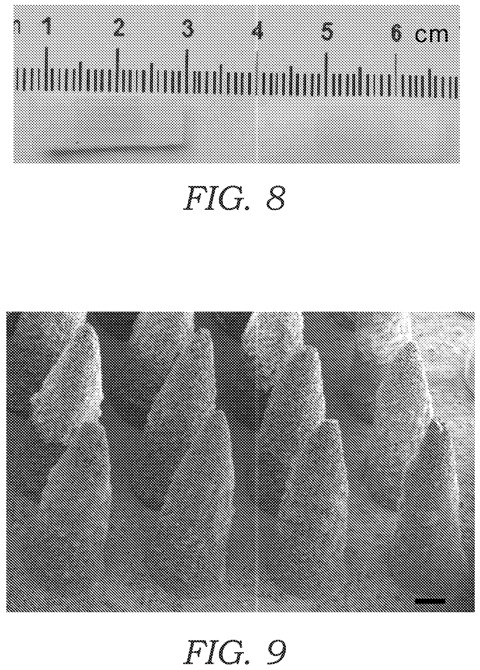

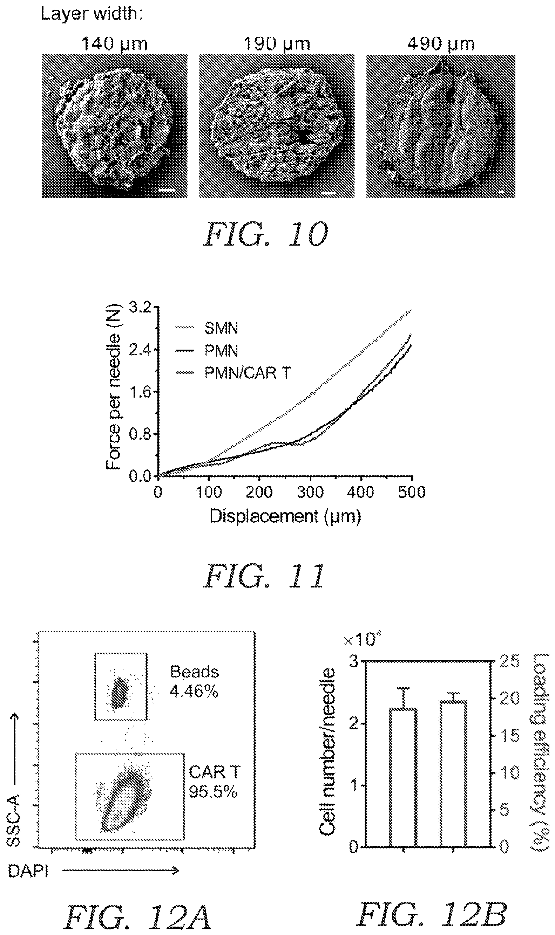

The porous microneedle patch 10 was fabricated by molding and polymerizing the mixture of methacryloyl chloride modified 4-arm-poly (lactic-co-glycolic acid) (4-arm-PLGA, FIG. 6), triethylene glycol diacetate along with CaCO₃ microparticles that range in size from about 2 µm to about 11 µm (most ranging from 6-8 µm) as seen in FIGS. 7A-7B. The size of the sacrificial microparticles should be on the micrometer scale and large enough to form pores 28 that can accommodate cells 30. Generally, this means that the sacrificial microparticles should have a diameter at or above around 5 µm. The 15 × 15 conical microneedle 14 has a base radius of 250 µm with 200 µm spacing, and up to 1500 µm in height (FIG. 1B). Subsequently, the microneedle patch 10 was swelled (FIG. 8), and CaCO₃ microparticles were etched in hydrogen chloride dioxane solution. SEM images revealed a jagged and porous surface formed after etching (FIG. 2D, FIG. 9). SEM images of the cross section of the porous microneedle patch 10 further demonstrated the pore 28 formation by etching the CaCO₃ microparticles (FIG. 2E, FIG. 10). In addition, the size of the pores 28 ranging 5 - 20 µm (average diameter), which is sufficient for loading CAR T cells 30 (5 - 10 µm). The porous microneedle 14 exhibited a slight weaker mechanical strength as the unetched microneedle, with a failure force of 2.4 N at 500 µm displacement, which can be attributed to the mechanical strength enhancement of the crosslinked structure and to the high molecular PLGA present in the formulation (FIG. 11). CAR T cells 30 were loaded into the microneedle 14 under vacuum (100 mbar) and bubble formation was observed during this process, no significant changes in mechanical force were detected post-loading the CAR T cells 30 (FIG. 11).

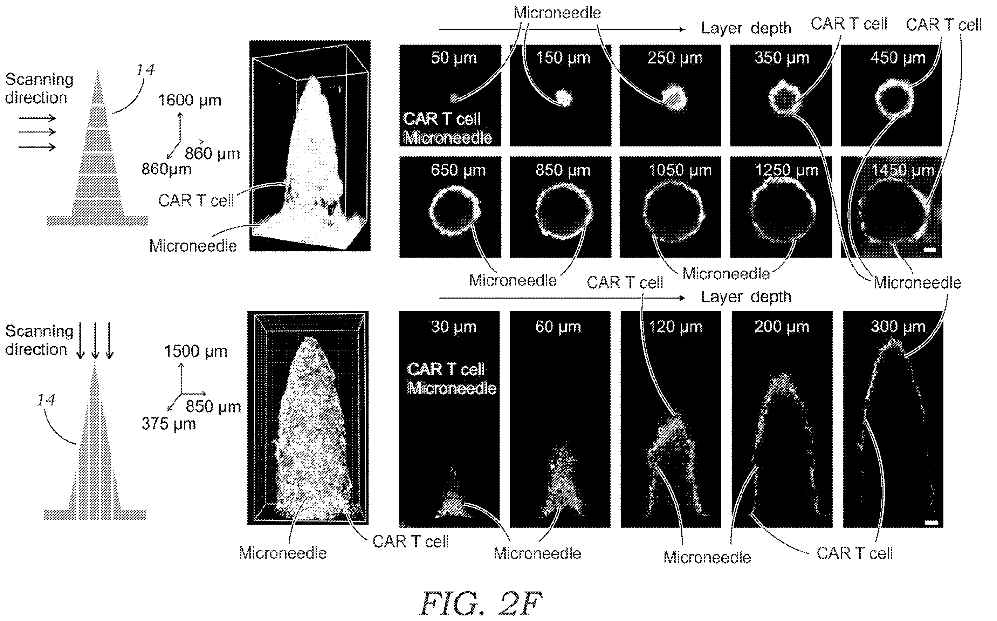

Chondroitin sulfate proteoglycan-4 (CSPG4) is overexpressed by multiple types of solid tumors including melanoma, breast cancer and mesothelioma. CSPG4-specific CAR T cells 30 (CAR CSPG4⁺ T cells) were loaded into a porous microneedle patch 10 and evaluated using a reconstructed 3D confocal image. CAR T cells resided in the interior space of the microneedle tip through a layer-by-layer scanning of the porous microneedle patch 10 with CLSM and 3D reconstruction (FIG. 2F). Quantitatively, each microneedle 14 was loaded with approximately 22,000 cells 30 as calculated by flow cytometry and counting beads (FIGS. 12A, 12B). Approximately 20% loading efficiency was achieved when the porous microneedle patch 10 was incubated with CAR T cells 30 at a density of 10⁷ cells/mL (FIGS. 12A, 12B).

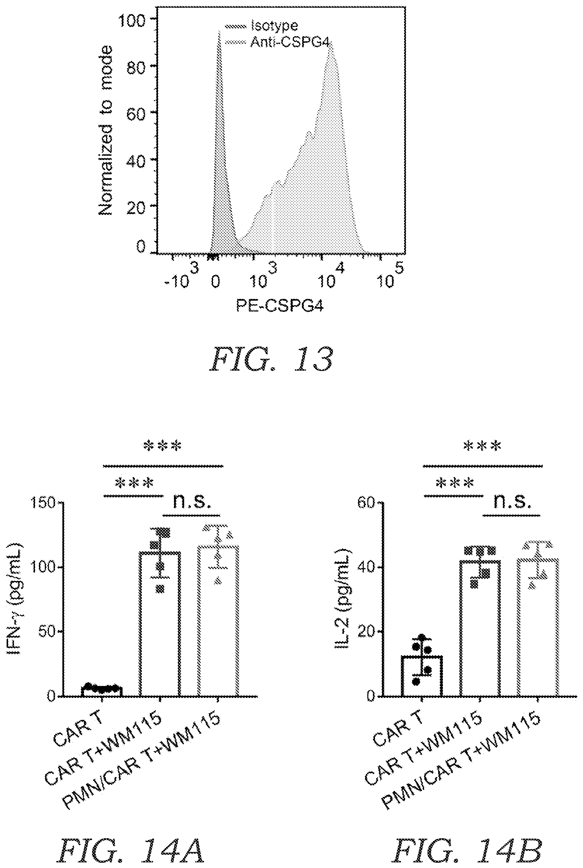

The biological effects of CAR T cells 30 loaded into the porous microneedle patch 10 was compared to those of free CAR T cells by analyzing cell proliferation, cytokine release and cytotoxic activity. CAR T cells labelled with carboxyfluorescein succinimidyl ester (CFSE) were cocultured with CSPG4 expressing human melanoma cancer cells (WM115, FIG. 13) for three days. CSFE dilution indicating cell division was observed in both CAR T cells 30 loaded into the porous microneedle patch 10 and free CAR T cells (FIG. 3A and 3B). Similarly, no differences were observed in interleukin-2 (IL-2) and interferon-y (IFN-y) release secretion (FIGS. 14B, 14B), and cytotoxic activity against CSPG4-expressing target (FIG. 3C) were noted between CAR T cells 30 loaded into the porous microneedle patch 10 and free CAR T cells, which demonstrated that high vigor remained of CAR T cells 30 loaded in the porous microneedle patch 10.

It was hypothesized that the array structure of the porous microneedle patch 10 can cover an extensive area and thus promotes wider distribution of CAR T cells 30 as compared to single needle-mediated injection of CAR T cells. Therefore, a 3D Matrigel model (cylinder, radius × height; 3.2 mm × 1.5 mm) was developed containing WM115 cancer cells to mimic the solid tumor *in vitro* (FIG. 3D). The mapping of CAR T cells 30 was determined with the 3D Matrigel model through dropwise (left in FIG. 3D), intra-gel injection (middle in FIG. 3D), and porous microneedle patch 10 (right in FIG. 3D) delivery. After three days of incubation, CAR T cell 30 delivery mediated by the porous microneedle patch 10 resulted in a homogenous mapping of the CAR T cells 30, while the CAR T cells administrated by dropwise and intra-gel injection were restricted to a confined space (FIG. 3D). Furthermore, approximately 2-fold higher level of IFN-y (FIG. 3E) and IL-2 (FIG. 3F) were secreted by CAR T cells 30 loaded into the porous microneedle patch 10 as compared to the direct injection or deposed on the surface of the mimic tumor model further confirming better distribution of CAR T cell 30 via porous microneedle patch 10 delivery.

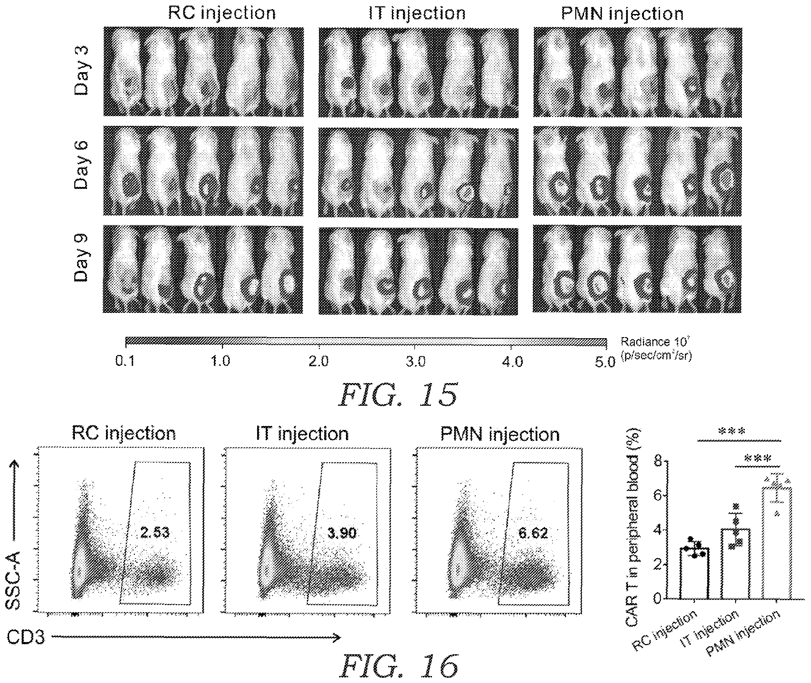

The antitumor efficacy of CAR T cells 30 delivered with a microneedle patch 10 *in vivo* was then evaluated. First, the intratumoral distribution of DiO pre-labelled CAR T cells 30 delivered through intratumoral injection and microneedle patch-mediated delivery in the WM115 melanoma tumor model in NOD.Cg-Prkdc^{scid} Il2rg^{tm1Wjl}/SzJ (NSG) mice was compared. Twenty hours post-application, CAR T cells were restricted to the applied region when CAR T cells were inoculated intratumoral (FIG. 4A). In contrast, CAR T cells 30 delivered via the microneedle patch 10 showed more prominent infiltration (FIG. 4B), providing more opportunity of encountering the cancer cells to initial CAR T cells 30 activation and killing. The proliferation and persistence of CAR T cells 30 in the post-surgery model in WM115 melanoma-bearing mice was then tested. After removing 90% of the tumor, firefly luciferase-labelled CAR T cells 30 *via* resection cavity injection at the surgical site, single needle intratumoral injection and porous microneedle-assisted injection, respectively. The proliferation of CAR T cells 30 was monitored using an *in vivo* imaging instrument (IVIS) spectrum system. As shown in FIG. 3D and FIG. 15, bioluminescence of free CAR-T cells 30 increased rapidly after implantation with the porous microneedle patch 10, and the quantitative analysis of bioluminescence signals of CAR T cells 30 demonstrated that microneedle patch-based delivery promoted 3-fold increases of bioluminescence signals as compared to other treatments (FIGS. 4C-4E). In parallel, porous microneedle-based delivery of CAR T cells 30 caused increased local expression of IL-2 and IFN-y (FIG. 4F). Increased bioluminescence of CAR T cells 30 corresponded to CAR T cell 30 expansion in response to tumor cells because at day 12 after treatment the number of CAR T cells 30 (CD3⁺) in the porous microneedle-treated mice was higher as compared to control groups (FIG. 4G, 4H). Of note, local delivery of CAR T cells 30 via the porous microneedle patch 10 caused systemic distribution of the CAR T cells 30 because these cells were detected in the peripheral blood (FIG. 16).

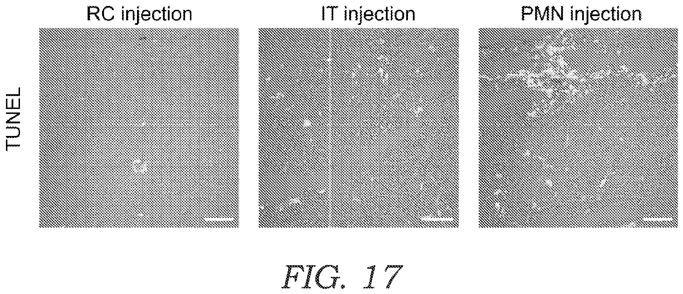

The CAR T cell delivery strategy was then investigated when combined with surgical tumor excision and whether this could benefit solid tumor treatment. Firefly luciferase-labelled WM115 cells were engrafted subcutaneously in NSG mice. Upon partial tumor resection, CAR T cells 30 were delivered directly in the resection cavity injection or intratumoral injection or via porous microneedle patch 10 application. The bioluminescence signal from the tumors and growth kinetics of the tumors significantly decreased in the mice received CAR T cell 30 delivery by the porous microneedle patch 10 compared with deposition and intratumoral injection (FIG. 5A-5D). The mean bioluminescence signal intensity of the tumor *via* porous microneedle-delivered CAR T cell treatment was only 5% of that received resection cavity injection, and 13% of that received intratumoral injection. The tumor weight (FIG. 5E) and size of (FIG. 5F) the tumors (as seen in images) further revealed the augment antitumor efficacy of the porous microneedle patch 10 for CAR T application. PMN/CAR T treated group showed higher cancer cell death in the TUNEL assay (FIG. 17), and more CD8⁺ and CD4⁺ T cells were detected under immunofluorescence staining (FIG. 5G). Taken together, CAR T cell 30 delivery with the porous microneedle patch 10 outperformed deposition administration and intratumoral injection methods.

Overall, the porous microneedle patch 10 offers a multipoint, scattered delivery of CAR T cells 30 seeding that promotes enhanced T cell infiltration within the tumor bed by overcoming poor T cell biodistribution caused by physical barriers in solid tumors. The porous microneedle patch 10 can be applied to the resection cavity post-surgery to prevent local tumor recurrence and metastatic dissemination and offer a platform for living cell delivery targeting a variety of diseases.

### Materials and Methods:

Linear poly(lactic-co-glycolic acid) (PLGA, lactic: glycolic 75:25, WM 76,000-115,000), methacryloyl chloride, triethylamine (TEA), tin(II) 2-ethylhexanoate, hydrogen chloride solution, 4.0 M in dioxane (HCl/Hexane) were purchased from Sigma Aldrich. Lactic (LA), glycolic (GA), pentaerythritol, azobisisobutyronitrile (AIBN), triethylene glycol diacetate (TEGDA) were purchased from TCI America. Calcium carbonate microparticle (~ 6 µm) were obtained from EggTech Ltd. (Canada).

The WM115 human melanoma cells were maintained in Dulbecco's Modified Eagle's Medium (DMEM, Gibco) supplemented with 10% fetal bovine serum (Invitrogen), 100 U/mL penicillin (Invitrogen) and 100 U/mL streptomycin (Invitrogen). Human T cells engineered with chondroitin sulfate proteoglycan 4 chimeric antigen receptors (CSPG4 CAR T) were cultured in complete medium containing 45% RPMI 1640 medium (Gibco) and 45% Click's medium (Irvine Scientific) with 10 % FBS (HyClone), 2 mmol/L GlutaMAX (Gibco), human recombinant interleukin 7 (5 ng/mL, Pepro Tech Inc) and human recombinant interleukin 15 (10 ng/mL, Pepro Tech Inc). Cells were cultured in an incubator (Thermo Fisher Scientific) at 37 °C under an atmosphere of 5% CO₂ and 90% relative humidity.

### Synthesis of 4-arm-PLGA-Acry

The LA (7.5 g), GA (2.5 g), pentaerythritol (0.14 g), and tin(II) 2-ethylhexanoate (10.0 mg) were mixed in a 25 mL flask bottle and heated to 130 °C in oil bath with stirring for 8 h. The product was dissolved with DMF and dialyzed (Cut WM 3,000 Da) for two days in DMF. The 4-arm-PLGA was further precipitated in water as white solid. The 4-arm-PLGA (5.0 g) was dissolved in dichloromethane mixed with TEA (1.0 g), and placed into an ice bath. The methacryloyl chloride (1.0 g) was dissolved in dichloromethane and added to the PLGA solution by drops, eight hours later, the product was purified by dialysis in DMF for another two days. The final product was obtained by precipitation in water as a white solid. ¹H NMR (400 M Hz, CDCl₃, ppm): δ 6.20 (s, 4H), 5.63 (s, 4H), 5.02-5.32 (m, 93H), 4.56-4.94 (m, 74H), 4.17 (s, 8H), 1.95 (s, 12H), 1.35-1.75 (m, 300H). ¹H NMR (FIG. 6) indicated the molecular weight of the 4-arm-PLGA-Acry as 10,000 Da, and the efficiency of the methacryloyl chloride modification was close to 100 %.

### Fabrication of the porous microneedle array patch

The 4-arm-PLGA-Acry was dissolved in dioxane with a final concentration of 500 mg/mL, AIBN was dissolved in Dioxane with a concentration of 100 mg/mL, and the linear PLGA was dissolved in Dioxane with a concentration of 200 mg/mL. Then, 300 mg of 4-arm-PLGA-Acry, 150 mg of TEGDA, 10 mg of AIBN, 20 mg of PLGA, and 90 mg of CaCO₃ microparticles were mixed and added to a polydimethylsiloxane (PDMS) micromold (Blueacre Technology Ltd.) with Dioxane pre-filled into the needle. Four hours later, the microneedle 14 was crosslinked overnight at 90 °C overnight before peeling off the microneedle patch 10. The microneedle patch 10 was placed in HCl/Hexane solution for two hours for swelling, and then water was added to initiate the reaction between HCl and CaCO₃ accompanied by CO₂ bubbles generated. Finally, the porous microneedle patch 10 was treated with plasma to generate a hydrophilic surface. The morphology of the porous microneedle patch was characterized by a scanning electron microscopy (SEM, ZEISS Supra 40VP).

### Loading CAR T cells into the porous microneedle patch

CAR T cells 30 were harvested from the medium and concentrated at 1×10⁷ cells/mL, and the porous microneedle patch 10 was placed into the CAR T cells 30 suspension under vacuum (100 mbar) for 20 min. Then the porous microneedle patch 10 was removed and the residual number of CAR T cells 30 were counted by flow cytometry with counting beads (Invitrogen) as an internal standard to determine the loading efficiency. For the observation of CAR T cells 30 loaded into the porous microneedle patch 10, CAR T cells 30 were incubated with CFSE (Thermo Fisher, cat. # C34554) for 10 min, then wash twice with PBS. The porous microneedle patch 10 was labelled with Rhodamine B by adding the dye when preparing the porous microneedle patch 10. After loading CAR T cells 30 into the porous microneedle patch 10 as introduced above, the porous microneedle patch 10 was placed in a glass-bottom dish and fixed with a fluorescent mounting medium (Thermo Fisher, cat. # TA-030-FM). The microdistribution of CAR T cells 30 on the porous microneedle patch 10 was detected by confocal laser scanning microscopy (CLSM, LS880, ZESSI) and reconstructed into 3D graphs with Imaris software.

### Mechanical strength test

The mechanical strength of microneedles 14 (before etching, after etching, and after loading with CAR T cells 30) was determined by pressing a stainless-steel plate against the porous microneedle patch 10 on an Instron tensile testing machine. The initial gauge was 2.00 mm between the tips 16 of microneedle (MN) 14 and the plate, with 10.00 N as the load cell capacity. The plate approaching MNs speed was set as 0.1 mm/s.

### Function and viability of the CAR T cells in vitro after loaded with microneedle patch

Luciferase expressing WM115 cells (WM115-Luc, 1×10⁵ cells/well) were incubated with CAR T cells or microneedle loaded CAR T cells 30 (MN@CAR T, 1×10⁵ cells/well), respectively in an ultralow adherent 24-well plate with complete medium. Three days later, the viability of WM115 cells, which is an indicator of the function of the CAR T cells 30, was evaluated by IVIS^{®} Spectrum *in vivo* imaging system (PerkinElmer) in the presence of luciferase substrate. In addition, the supernatant of the co-incubation medium was collected by centrifugation (1000 g, 10 min, 4 °C) to determine the cytokine secretion level of the CAR T cells 30. Human IL-2 and human IFN-γ secreted by CAR T cells 30 were measured by enzyme-linked immunosorbent assay (ELISA, IL-2, Invitrogen, cat. # 88-7025-22; IFN-γ, cat. # 88-7316-22) according to the manufacturer's instructions.

For the *in vitro* CAR T cells proliferation evaluation, CAR T cells 30 (1×10⁶) were incubated in the dark with CFSE (final concentration of 1 µM) for 10 min at room temperature. Excess CFSE was washed three times with complete medium before loading the labelled CAR T cell 30 into the microneedle 14 introduced previously. WM115 cells (1×10⁵ cells/well) and CAR T cells or porous microneedle patch-loaded CAR T cells 30 (1×10⁵ cells/well) were co-cultured in an ultralow adherent 24-well plate for three days with complete medium. The T cells 30 were stained with PE-CD3 (Biolegend, cat. no. 300308, clone HIT3a), and CAR T cells proliferation was detected by flow cytometry (BD LSRFortessa).

### Distribution of CAR T cells and WM115 cells in 3D Matrigel model in vitro

The CFSE labelled WM115 cells (1×10⁶ cells/mL) were dispersed in 1 mL phenol red-free DMEM medium (containing 10% FBS) and mixed with 1 mL phenol red-free Matrigel (Corning) by pipetting. The mixture was transferred to a glass-bottom 96-well plate (50 µL/well) and incubated at 37 °C to form a gel within 24 hours. DIO labelled CAR T cells 30 were administrated to the gels by dropwise, injection, and microneedle delivery, followed by incubation for three days. The distribution of CAR T cells 30 and WM 115 cells were observed with CLSM (LS880, ZESSI).

### Xenograft WM115 melanoma tumor generation

The NOD.Cg-Prkdc^{scid} Il2rg^{tm1Wjl}/SzJ (NSG) mice (female, 6 - 8 weeks) were purchased from the Jackson laboratory. All animal experiments were performed in compliance with an animal study protocol approved by the Institutional Animal Care and Use Committee at University of California, Los Angeles. WM115 (5×10⁶) cells were injected into the NSG mice subcutaneously. The xenograft melanoma tumor grows to the size of 100 mm³ within one-month after the inoculation of the WM115 cell. Before a treatment or other tumor-associated experiments, the solid tumor was resected, leaving ~10% tumor to mimic residual micro tumor after surgery.

### Distribution of CAR T cells in tumor

CAR T cells 30 were labelled with DIO according to the manufacturer's instructions, then the CAR T cells 30 applied to the WM115 melanoma tumor by intratumor injection or microneedle delivery. 24 hours later, the tumors were harvested and embedded in OCT to obtain frozen sections, the slides of the tumor section were observed with CLSM (LS880, ZESSI) to display the distribution of CAR T cells 30 in tumor.

### CAR T cell proliferation in vivo

CAR T cells 30 (1×10⁶) with luciferase expression were administrated to the residual tumor by subcutaneous injection, intratumoral injection or porous microneedle patch injection. After application, the bioluminescence signals were recorded on day 0, day 3, day 6, day 9 and day 12 with IVIS (Perkin). Signal analysis was performed by Living Image Software. Twelve days after application, the tumors were harvested, digested into single cells, and labelled with anti-human CD3 antibody (Biolegend, cat. no. 300308, clone HIT3a) to count the number of CAR T cells 30 by flow cytometry. The cytokines secretion by CAR T cells 30, including human IL-2 and human IFN-γ, were analyzed with ELISA (Invitrogen) according to the manufacturer's instructions.

### In vivo anti-tumor activity

WM115 cells (5×10⁶) with luciferase expression were injected into the NSG mice subcutaneously. 90 % of the tumor was removed surgically when reached 100 mm³. CAR T cells 30 (1×10⁶) were administrated *via* subcutaneous injection, intratumoral injection, and porous microneedle patch injection to the tumor sites. After CAR T cells 30 application, the bioluminescence signals were recorded on day 0, day 3, day 6, day 9, day 12 and day 15 with IVIS (Perkin) (see e.g., FIG. 15). The analysis of signals was performed by Living Image Software. After the treatment, the mice were sacrificed, the tumor weight and the images of the tumors were recorded.

### Immunofluorescence staining

Tumors were harvested from the mice and froze in the optimal cutting temperature (OCT) medium before cutting *via* a cryotome. 20 µg/mL Alexa Fluor^{®} 594 anti-human CD8a antibody (Biolegend, cat no. 100758) and Alexa Fluor^{®} 488 anti-human CD4 antibody (Biolegend, cat no. 317408) was used to staining the tumor slices overnight at 4 °C. The tumor cell apoptosis was analyzed with terminal deoxynucleotidyl transferase dUTP nick end labeling (TUNEL) assay kit following the standard protocol.

### Statistical analysis

All results are presented as the mean ± standard deviation (SD), as indicated. Tukey post-hoc tests and one-way ANOVA were used for multiple comparisons. Survival benefit was determined using a log-rank (mantel-cox) test. All statistical analyses were carried out with Prism software package (PRISM 5.0; GraphPad Software, 2007). The threshold for statistical significance was *P*<0.05.

## Claims

1. A porous microneedle patch (10) for the localized delivery of live chimeric antigen receptor T (CAR T) cells to living tissue (100) comprising:
a base (12) having a plurality of microneedles (14) extending away from the surface of the base (12), wherein the plurality of microneedles (14) comprise crosslinked methacryloyl chloride modified multi-arm-poly (lactic-co-glycolic acid)(PLGA) having pores formed therein, wherein the pores contain live CAR Tcells (30) therein.

2. The porous microneedle patch (10) of claim 1, wherein the pores have an average diameter within the size range of about 5-20 µm.

3. The porous microneedle patch (10) of claim 1, wherein the plurality of microneedles have a length of less than about 2.0 mm.

4. A method of manufacturing a porous microneedle patch (10) for the localized delivery of living cells to living tissue (100) comprising:
providing a mold containing a plurality of needle-shaped cavities therein;
applying a solution containing a polymerizable polymer comprising methacryloyl chloride modified multi-arm-PLGA, an initiator or crosslinking agent, and sacrificial microparticles to the mold;
crosslinking the solution to create a patch;
removing the patch from the mold and placing the same in a swelling solution;
placing the swelled patch in an etching solution to remove the sacrificial microparticles and form pores in the porous microneedle patch; and
loading cells (30) into the pores of the porous microneedle patch.

5. The method of claim 4, wherein the solution containing the polymerizable polymer further comprises, triethylene glycol diacetate, and azobisisobutyronitrile (AIBN).

6. The method of claim 5, wherein the solution containing the polymerizable polymer further comprises linear PLGA.

7. The method of claim 4, wherein the sacrificial microparticles comprise CaCO₃ microparticles.

8. The method of claim 4, wherein the sacrificial microparticles comprise one of: magnesium carbonate, manganese carbonate, barium carbonate, sodium chloride, and calcium chloride.

9. The method of claim 4, wherein the cells comprise CAR T cells, pluripotent stem cells, or dendritic cells.

10. The method of claim 4, wherein swelling solution comprises a hydrogen chloride/hexane solution.

11. The method of claim 4, wherein the live cells are loaded into the pores of the porous microneedle patch by exposing the porous microneedle patch to a solution containing live cells and optionally applying a vacuum to the porous microneedle patch and solution containing the live cells.

## Patentansprüche

1. Ein poröser Mikronadelpatch (10) zur lokalisierten Abgabe von lebenden chimären Antigenrezeptor-T (CAR T) Zellen an lebendes Gewebe (100), umfassend:
eine Basis (12) mit einer Vielzahl von Mikronadeln (14), die sich von der Oberfläche der Basis (12) weg erstrecken, wobei die Vielzahl von Mikronadeln (14) vernetztes Methacryloylchlorid modifiziertes Multi-Arm-Poly(lactid-*co*-glycolid)( PLGA) umfassen und Poren darin gebildet sind, wobei die Poren lebende CAR T Zellen (30) enthalten.

2. Der poröse Mikronadelpatch (10) nach Anspruch 1, wobei die Poren einen durchschnittlichen Durchmesser im Größenbereich von etwa 5-20 µm haben.

3. Der poröse Mikronadelpatch (10) nach Anspruch 1, wobei die Vielzahl von Mikronadeln eine Länge von weniger als etwa 2,0 mm haben.

4. Ein Verfahren zur Herstellung eines porösen Mikronadelpatches (10) zur lokalisierten Abgabe von lebenden Zellen an lebendes Gewebe (100), umfassend:
Bereitstellung einer Form mit einer Vielzahl von nadelförmigen Hohlräumen darin;
Aufbringen einer Lösung, die ein polymerisierbares Polymer, bestehend aus Methacryloylchlorid modifiziertem Multi-Arm-PLGA, einen Initiator oder Vernetzungsagens und opferbare Mikropartikel enthält, auf die Form;
Vernetzung der Lösung, um einen Patch zu erzeugen;
Entfernung des Patches aus der Form und Platzierung desselben in einer Quelllösung;
Platzierung des gequollenen Patches in einer Ätzlösung, um die opferbaren Mikropartikel zu entfernen und Poren im porösen Mikronadelpatch zu bilden; und
Beladung der Zellen (30) in die Poren des porösen Mikronadelpatches.

5. Das Verfahren nach Anspruch 4, wobei die Lösung, die das polymerisierbare Polymer enthält, zusätzlich Triethylenglykoldiacetat und Azobisisobutyronitril (AIBN) enthält.

6. Das Verfahren nach Anspruch 5, wobei die Lösung, die das polymerisierbare Polymer enthält, zusätzlich lineares PLGA enthält.

7. Das Verfahren nach Anspruch 4, wobei die opferbaren Mikropartikel CaCO₃-Mikropartikel umfassen.

8. Das Verfahren nach Anspruch 4, wobei die opferbaren Mikropartikel eines der folgenden umfassen: Magnesiumcarbonat, Mangancarbonat, Bariumcarbonat, Natriumchlorid und Calciumchlorid.

9. Das Verfahren nach Anspruch 4, wobei die Zellen CAR T Zellen, pluripotente Stammzellen oder dendritische Zellen umfassen.

10. Das Verfahren nach Anspruch 4, wobei die Quelllösung eine Wasserstoffchlorid/Hexan-Lösung umfasst.

11. Das Verfahren nach Anspruch 4, wobei die lebenden Zellen in die Poren des porösen Mikronadelpatches durch Exposition des porösen Mikronadelpatches gegenüber einer Lösung, die lebende Zellen enthält, und optional durch Anwendung eines Vakuums auf den porösen Mikronadelpatch und die Lösung, die die lebenden Zellen enthält, geladen werden.

## Revendications

1. Un patch de micro-aiguilles poreuses (10) pour la délivrance localisée de cellules vivantes de récepteur antigénique chimérique T (CAR T) à un tissu vivant (100) comprenant :
une base (12) ayant une pluralité de micro-aiguilles (14) s'étendant loin de la surface de la base (12), où la pluralité de micro-aiguilles (14) comprend du chlorure de méthacryloyle modifié multi-bras-poly (acide lactique-co-glycolique) (PLGA) réticulé ayant des pores formés dans ceux-ci, où les pores contiennent des cellules CAR T vivantes (30) à l'intérieur.

2. Le patch de micro-aiguilles poreuses (10) de la revendication 1, où les pores ont un diamètre moyen dans la plage de taille d'environ 5-20 µm.

3. Le patch de micro-aiguilles poreuses (10) de la revendication 1, où la pluralité de micro-aiguilles ont une longueur de moins d'environ 2.0 mm.

4. Un procédé de fabrication d'un patch de micro-aiguilles poreuses (10) pour la délivrance localisée de cellules vivantes à un tissu vivant (100) comprenant :
la fourniture d'un moule contenant une pluralité de cavités en forme de aiguilles ;
l'application d'une solution contenant un polymère polymérisable comprenant du chlorure de méthacryloyle modifié multi-bras-PLGA, un initiateur ou agent réticulant, et des microparticules sacrificielles au moule ;
la réticulation de la solution pour créer un patch ;
l'enlèvement du patch du moule et le placement du même dans une solution de gonflement ;
le placement du patch gonflé dans une solution de gravure pour enlever les microparticules sacrificielles et former des pores dans le patch de micro-aiguilles poreuses ; et
le chargement de cellules (30) dans les pores du patch de micro-aiguilles poreuses.

5. Le procédé de la revendication 4, où la solution contenant le polymère polymérisable comprend également, du diacétate de triéthylène glycol, et de l'azobisisobutyronitrile (AIBN).

6. Le procédé de la revendication 5, où la solution contenant le polymère polymérisable comprend également du PLGA linéaire.

7. Le procédé de la revendication 4, où les microparticules sacrificielles comprennent des microparticules de CaCO3.

8. Le procédé de la revendication 4, où les microparticules sacrificielles comprennent un de : carbonate de magnésium, carbonate de manganèse, carbonate de baryum, chlorure de sodium, et chlorure de calcium.

9. Le procédé de la revendication 4, où les cellules comprennent des cellules CAR T, des cellules souches pluripotentes, ou des cellules dendritiques.

10. Le procédé de la revendication 4, où la solution de gonflement comprend une solution d'hydrogène chlorure/hexane.

11. Le procédé de la revendication 4, où les cellules vivantes sont chargées dans les pores du patch de micro-aiguilles poreuses en exposant le patch de micro-aiguilles poreuses à une solution contenant des cellules vivantes et en appliquant éventuellement un vide au patch de micro-aiguilles poreuses et à la solution contenant les cellules vivantes.
